(19) Europäisches Patentamt
European Patent Office
Office européen des brevets

(11) Veröffentlichungsnummer: **0 435 043 A1**

(12) **EUROPÄISCHE PATENTANMELDUNG**

(21) Anmeldenummer: **90123609.1**

(51) Int. Cl.5: **C07D 405/12**, A01N 43/36

(22) Anmeldetag: **08.12.90**

(30) Priorität: **23.12.89 DE 3942895**
**10.02.90 DE 4004035**

(43) Veröffentlichungstag der Anmeldung:
**03.07.91 Patentblatt 91/27**

(84) Benannte Vertragsstaaten:
**BE CH DE FR GB IT LI NL**

(71) Anmelder: **BAYER AG**

**W-5090 Leverkusen 1 Bayerwerk(DE)**

(72) Erfinder: **Wollweber, Detlef, Dr.**
**Paul-Ehrlich-Strasse 17**
**W-5600 Wuppertal 1(DE)**
Erfinder: **Krämer, Wolfgang, Dr.**
**Rosenkranz 25**
**W-5093 Burscheid 2(DE)**
Erfinder: **Brandes, Wilhelm, Dr.**
**Eichendorffstrasse 3**
**W-5653 Leichlingen 1(DE)**
Erfinder: **Dutzmann, Stefan, Dr.**
**Leinenweberweg 33**
**W-4000 Düsseldorf 13(DE)**
Erfinder: **Paulus, Wilfried, Dr.**
**Deswatinesstrasse 90**
**W-4150 Krefeld 1(DE)**

(54) **Substituerte 1-Aminomethyl-3-aryl-4-cyano-pyrrole.**

(57) Neue substituierte 1-Aminomethyl-3-aryl-4-cyano-pyrrole der Formel (I)

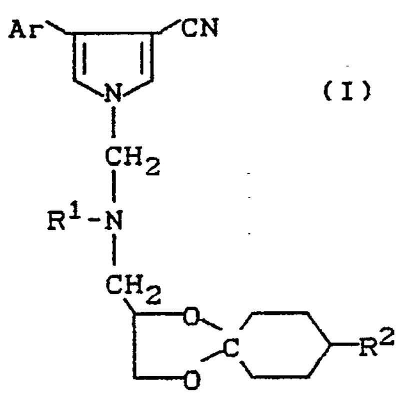

(I)

in welcher
die Substituenten die in der Beschreibung angegebenen Bedeutungen haben, ihre Herstellung und Verwendung im Pflanzenschutz und Materialschutz.

Die neuen Verbindungen sind durch die Formel (I) allgemein definiert und können nach Analogieverfahren hergestellt werden, z.B. aus geeigneten 3-Aryl-4-cyano-pyrrolen und Formaldehyd und Aminen bzw. nur Aminen.

EP 0 435 043 A1

## SUBSTITUIERTE 1-AMINOMETHYL-3-ARYL-4-CYANO-PYRROLE

Die Erfindung betrifft neue substituierte 1-Aminomethyl-3-aryl-4-cyano-pyrrole, mehrere Verfahren zu ihrer Herstellung, ihre Verwendung zur Bekämpfung von Schädlingen, insbesondere pflanzenschädigende Pilze sowie ihre Verwendung als Mikrobizide im Materialschutz.

Es ist bereits bekannt, daß substituierte 1-Aminomethyl-3-aryl-4-cyano-pyrrole, wie beispielsweise 4-Cyano-3-(2,3-dichlorphenyl)-1-(N-cyclohexyl-N-tetrahydrofurfurylaminomethyl)-pyrrol oder 4-Cyano-3-(2,3-dichlorphenyl)-pyrrol oder 4-Cyano-3-(2,3-dichlorphenyl)-1-(N,N-dimethylaminomethyl)-pyrrol gute fungizide Eigenschaften besitzen (vgl. unter anderem z.B. EP-OS 281 731, EP-OS 174 910, EP-OS 133 247, EP-OS 182 738, EP-OS 206 999 oder EP-OS 310 558).

Weiterhin ist bekannt, daß bestimmte Sulfenamide, wie beispielsweise das N,N-Dimethyl-N'-phenyl-N'-(fluordichlormethylsulfenyl)-sulfamid insbesondere gegen Botrytis-Arten hervorragend wirksam sind (vgl. z.B. K.H. Büchel "Pflanzenschutz und Schädlingsbekämpfung", S. 141. Thieme Verlag Stuttgart 1977).

Es ist weiter bereits bekannt, bestimmte Azolyl-methylamine, wie beispielsweise 1,2,4-Triazol-1-yl-methyl-di-n-octylamin als Wirkstoffe zur Bekämpfung von Mikroorganismen, die technische Materialien befallen, schädigen und zerstören können, einzusetzen (vgl. EP-OS 106 243). Azolyl-methylamine zählen zu den Formaldehyd-abspalten-den Verbindungen, d.h., daß ihre antimikrobielle Wirkung auf ihrer Fähigkeit, Formaldehyd abzuspalten beruht (Paulus, W., 1980. Formaldehyde releasing compounds and their utility as microbicides. Biodet. Proceed. of the IV. Internat. Symp., Pitman Publishing Ltd., London, S. 307-314).

Die Wirksamkeit dieser vorbekannten Verbindungen ist jedoch insbesondere bei niedrigen Aufwandmengen und Konzentrationen nicht in allen Anwendungsbereichen völlig zufriedenstellend.

Es wurden neue substituierte 1-Aminomethyl-3-aryl-4-cyano-pyrrole der allgemeinen Formel (I),

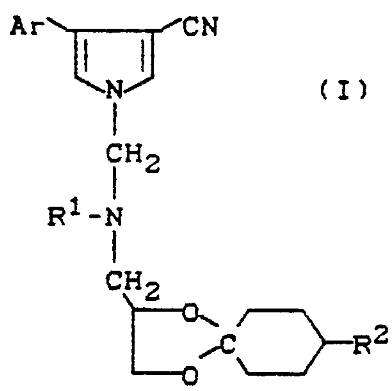

(I)

in welcher

Ar für gegebenenfalls substituiertes Phenyl steht,

$R^1$ für gegebenenfalls substituiertes Alkyl, für Alkenyl, Alkinyl oder Cycloalkyl oder für jeweils gegebenenfalls substituiertes Aralkyl oder Aryl steht und

$R^2$ für Alkyl oder für jeweils gegebenenfalls substituiertes Cycloalkylalkyl, Aralkyl, Cycloalkyl oder Aryl steht,

und deren Säureadditionssalze gefunden.

Die Verbindungen der Formel (I) können als geometrische und/oder optische Isomere oder Isomerengemische unterschiedlicher Zusammensetzung vorliegen. Sowohl die reinen Isomeren als auch die Isomerengemische werden erfindungsgemäß beansprucht, wenn von Verbindungen der Formel (I) die Rede ist.

Weiterhin wurde gefunden, daß man die neuen substituierten 1-Aminomethyl-3-aryl-4-cyano-pyrrole der allgemeinen Formel (I),

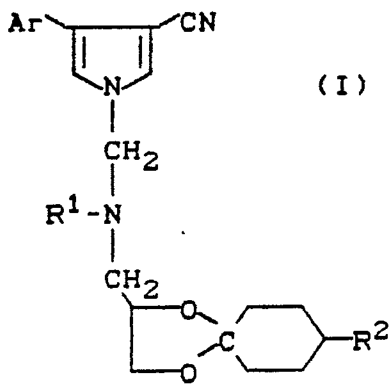

(I)

in welcher

Ar für gegebenenfalls substituiertes Phenyl steht,

$R^1$ für gegebenenfalls substituiertes Alkyl, für Alkenyl, Alkinyl oder Cycloalkyl oder für jeweils gegebenenfalls substituiertes Aralkyl oder Aryl steht und

$R^2$ für Alkyl oder für jeweils gegebenenfalls substituiertes Cycloalkylalkyl, Aralkyl, Cycloalkyl oder Aryl steht,

und deren Säureadditionssalze erhält, wenn man

(a) 3-Aryl-4-cyano-pyrrole der Formel (II),

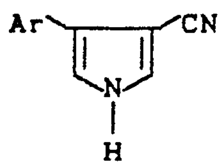

(II)

in welcher

Ar die oben angegebene Bedeutung hat,

mit Formaldehyd und Aminen der Formel (III),

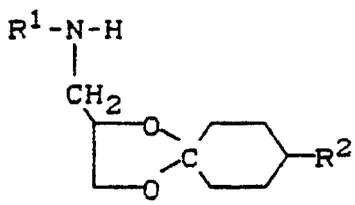

(III)

in welcher

$R^1$ und $R^2$ die oben angegebene Bedeutung haben,

gegebenenfalls in Gegenwart eines Verdünnungsmittels und gegebenenfalls in Gegenwart eines Reaktionshilfsmittels umsetzt, oder wenn man

(b) 3-Aryl-4-cyano-pyrrole der Formel (IV),

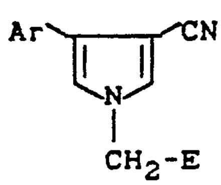

(IV)

in welcher

Ar die oben angegebene Bedeutung hat und

E für eine elektronenanziehende Abgangsgruppe steht,

mit Aminen der Formel (III),

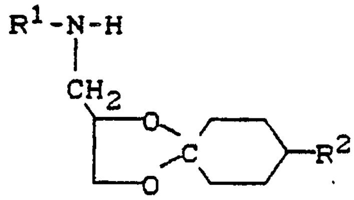

(III)

in welcher

$R^1$ und $R^2$ die oben angegebene Bedeutung haben,

gegebenenfalls in Gegenwart eines Verdünnungsmittels und gegebenenfalls in Gegenwart eines Säurebindemittels umsetzt und gegebenenfalls anschließend eine Säure addiert oder eine physikalische Trennmethode anschließt.

Schließlich wurde gefunden, daß die neuen substituierten 1-Amino-methyl-3-aryl-4-cyano-pyrrole der allgemeinen Formel (I) eine gute Wirkung gegen Schädlinge, insbesondere gegen pilzliche Schädlinge, besitzen.

Überraschenderweise zeigen die erfindungsgemäßen substituierten 1-Aminomethyl-3-aryl-4-cyano-pyrrole der allgemeinen Formel (I) eine bessere fungizide Wirksamkeit im Vergleich zu den aus dem Stand der Technik vorbekannten Sulfenamiden, wie beispielsweise dem N,N-Dimethyl-N'-phenyl-N'-(fluordichlormethylsulfenyl)-sulfamid oder den aus dem Stand der Technik vorbekannten 3-Aryl-pyrrolen, wie beispielsweise dem 4-Cyano-3-(2,3-dichlor-phenyl)-pyrrol oder dem 4-Cyano-3-(2,3-dichlorphenyl)-1-(N-cyclohexyl-N-furfurylaminomethyl)-pyrrol oder dem 4-Cyano-3-(2,3-dichlorphenyl)-1-(N,N-dimethyl-aminomethyl)-pyrrol, welche chemisch und/oder wirkungsmäßig naheliegende Verbindungen sind.

Die erfindungsgemäßen substituierten 1-Aminomethyl-3-aryl-4-cyano-pyrrole der allgemeinen Formel (I) sind überraschenderweise nicht nur wesentlich wirksamer als in anbetracht ihres Gehaltes an abspaltbarem Formaldehyd zu erwarten ist, sondern auch wirksamer als die aus dem Stand der Technik vorbekannten Azolylmethylamine, wie beispielsweise 1,2,4-Triazol-1-yl-methyl-di-n-octylamin, welches eine chemisch und/oder wirkungsmäßig naheliegende Verbindung ist (vgl. EP 106 243). Darüber hinaus verfügen die erfindungsgemäßen Verbindungen der Formel (I) über das breitere und ausgeglichenere Wirkungsspektrum, so daß sie mit Vorteil gegenüber dem Stand der Technik als Mikrobizide zum Schutz technischer Materialien, die ja von einer Vielzahl verschiedener Mikrobenarten befallen, geschädigt und zerstört werden können, anwendbar sind.

Die erfindungsgemäßen substituierten 1-Aminomethyl-3-aryl-4-cyano-pyrrole sind durch die Formel (I) allgemein definiert. Bevorzugt sind Verbindungen der Formel (I), bei welchen

Ar für gegebenenfalls einfach bis mehrfach, gleich oder verschieden substituiertes Phenyl steht, wobei als Substituenten infrage kommen: Halogen jeweils geradkettiges oder verzweigtes Alkyl, Alkoxy, Alkylthio, Halogenalkyl, Halogenalkoxy oder Halogenalkylthio mit jeweils 1 bis 4 Kohlenstoffatomen und gegebenenfalls 1 bis 9 gleichen oder verschiedenen Halogenatomen oder Difluormethylendioxy,

$R^1$ für gegebenenfalls substituiertes, geradkettiges oder verzweigtes Alkyl mit 1 bis 6 Kohlenstoffatomen steht, wobei als Substituenten infrage kommen: Cyano, Cycloalkyl mit 3 bis 7 Kohlenstoffatomen, jeweils geradkettiges oder verzweigtes Alkoxy, Alkylcarbonyl, Alkoxycarbonyl, Alkylaminocarbonyl, Dialkylaminocarbonyl, Alkylsulfinyl, Alkylsulfonyl oder Dialkylamino mit jeweils 1 bis 6 Kohlenstoffatomen in den einzelnen Alkylteilen; außerdem für jeweils geradkettiges oder verzweigtes Alkenyl oder Alkinyl mit jeweils 3 bis 6 Kohlenstoffatomen, für Cycloalkyl mit 3 bis 7 Kohlenstoffatomen oder für jeweils gegebenenfalls einfach bis mehrfach, gleich oder verschieden im Phenylteil substituiertes Phenyl oder Benzyl steht, wobei als Substituenten jeweils infrage kommen: Halogen, Cyano, jeweils geradkettiges oder verzweigtes Alkyl, Alkoxy, Halogenalkyl, Halogenalkoxy, Alkylcarbonyl oder Alkoxycarbonyl mit jeweils 1 bis 6 Kohlenstoffatomen in den einzelnen Alkylteilen und im Fall des Halogenalkyl- oder Halogenalkoxy-Restes mit jeweils 1 bis 9 gleichen oder verschiedenen Halogenatomen und

$R^2$ für geradkettiges oder verzweigtes Alkyl mit 1 bis 14 Kohlenstoffatomen, für jeweils gegebenenfalls einfach bis mehrfach, gleich oder verschieden durch Halogen und/oder geradkettiges oder verzweigtes Alkyl mit 1 bis 8 Kohlenstoffatomen substituiertes Cycloalkylalkyl oder Cycloalkyl mit jeweils 3 bis 7 Kohlenstoffatomen im Cycloalkylteil und gegebenenfalls 1 bis 6 Kohlenstoffatomen im geradkettigen oder verzweigten Alkylteil oder für jeweils gegebenenfalls einfach bis mehrfach,

gleich oder verschieden durch Halogen und/oder geradkettiges oder verzweigtes Alkyl mit 1 bis 8 Kohlenstoffatomen substituiertes Aralkyl oder Aryl mit jeweils 6 bis 10 Kohlenstoffatomen im Arylteil und gegebenenfalls 1 bis 6 Kohlenstoffastomen im geradkettigen oder verzweigten Alkylteil steht.

Besonders bevorzugt sind Verbindungen der Formel (I), bei welchen

Ar für gegebenenfalls ein- bis dreifach, gleich oder verschieden substituiertes Phenyl steht, wobei als Substituenten infrage kommen: Fluor, Chlor, Brom, Methyl, Ethyl, n- oder i-Propyl, n-, i-, s- oder t-Butyl, Methoxy, Ethoxy, Methylthio, Trifluormethyl, Trifluormethoxy, Trifluormethylthio oder Difluormethylendioxy,

$R^1$ für Methyl, Ethyl, n- oder i-Propyl, n-, i- oder s-Butyl, Pentyl oder Hexyl, für Cyclohexylmethyl oder durch 1 - 3 Methylgruppen substituiertes Cyclohexylmethyl steht, außerdem für Allyl, n-oder i-Butenyl, Propargyl, n- oder i-Butinyl, für Cyclopentyl, Cyclohexyl oder Cyclopropyl steht, wobei diese durch 1 - 3 Methylgruppen substituiert sein können oder für jeweils gegebenenfalls ein- bis dreifach, gleich oder verschieden im Phenylteil substituiertes Benzyl oder Phenyl steht, wobei als Substituenten infrage kommen: Fluor, Chlor, Brom, Cyano, Methyl, Ethyl, n- oder i-Propyl, n-, i- oder s-Butyl, Methoxy, Ethoxy, Trifluormethyl, Trifluormethoxy, Acetyl, Methoxycarbonyl oder Ethoxycarbonyl und

$R^2$ für geradkettiges oder verzweigtes Alkyl mit 1 bis 12 Kohlenstoffatomen, für gegebenenfalls ein- bis dreifach, gleich oder verschieden durch Methyl, Ethyl, n- oder i-Propyl, n-, i-, s- oder t-Butyl substituiertes Cyclohexyl, für im Cyclohexylteil gegebenenfalls ein- bis dreifach, gleich oder verschieden durch Methyl, Ethyl, n- oder i-Propyl, n-, i-, s- oder t-Butyl substituiertes Cyclohexylalkyl mit 1 bis 4 Kohlenstoffatomen im geradkettigen oder verzweigten Alkylteil, für gegebenenfalls ein- bis dreifach, gleich oder verschieden durch Methyl, Ethyl, n- oder i-Propyl, n-, i-, s-oder t-Butyl substituiertes Phenyl oder für im Phenylteil gegebenenfalls ein- bis dreifach, gleich oder verschieden durch Methyl, Ethyl, n- oder i-Propyl, n-, i-, s- oder t-Butyl substituiertes Phenylalkyl mit 1 bis 4 Kohlenstoffatomen im geradkettigen oder verzweigten Alkylteil steht.

Ganz besonders bevorzugt sind Verbindungen der Formel (I), bei welchen

Ar für dreifach, gleich oder verschieden durch Chlor, Fluor, Methyl, Trifluormethyl oder Trifluormethoxy substituiertes Phenyl steht,

$R^1$ für Ethyl, n- oder i-Propyl, n-, i- oder s-Butyl, n-Pentyl, n-Hexyl, Allyl, Propargyl, für Cyclohexyl, Cyclopentyl, Cyclohexylmethyl, Methylcyclohexyl, 4-Methylcyclohexylmethyl oder 3-Methylcyclohexylmethyl steht und

$R^2$ für Cyclohexyl, Phenyl oder einen der Reste

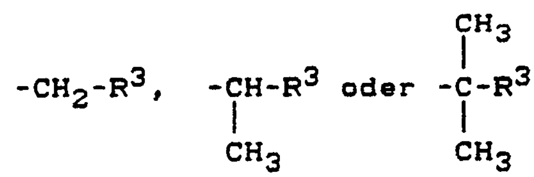

, oder

steht, wobei

$R^3$ jeweils für Wasserstoff, Methyl, Ethyl, n- oder i-Propyl, n-, i-, s- oder t-Butyl, Neopentyl, Cyclohexyl oder Phenyl steht.

Bevorzugte erfindungsgemäße Verbindungen sind auch Additionsprodukte aus Säuren und denjenigen substituierten 1-Aminomethyl-3-aryl-4-cyano-pyrrolen der Formel (I), in denen die Substituenten Ar, $R^1$ und $R^2$ die Bedeutungen haben, die bereits für diese Substituenten genannt wurden.

Zu den Säuren die addiert werden können und die zu pflanzenverträglichen Additionsprodukten führen, gehören vorzugsweise Halogenwasserstoffsäuren, wie z.B. Chlorwasserstoffsäure und Bromwasserstoffsäure, insbesondere Chlorwasserstoffsäure, ferner Phosphorsäure, Salpetersäure, mono-, bi- und trifunktionelle Carbonsäuren und Hydroxycarbonsäuren, wie z.B. Essigsäure, Maleinsäure, Bernsteinsäure, Fumarsäure, Weinsäure, Zitronensäure, Salicylsäure, Sorbinsäure und Milchsäure, Sulfonsäuren, wie p-Toluolsulfonsäure und 1,5-Naphthalindisulfonsäure sowie Saccharin oder Thiosaccharin.

Im einzelnen seien außer den bei den Herstellungsbeispielen genannten Verbindungen die folgenden 1-Aminomethyl-3-aryl-4-cyano-pyrrole der allgemeinen Formel (I) genannt:

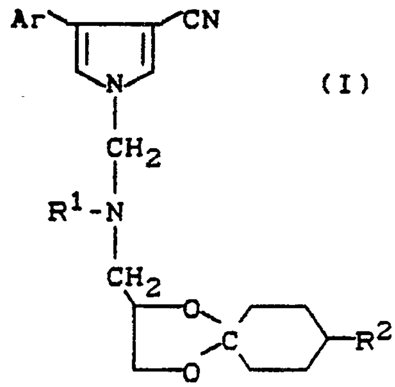

(I)

Tabelle 1:

| Ar | $R^1$ | $R^2$ |
|---|---|---|
| 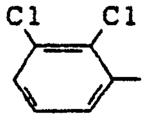 | $-C_2H_5$ | $-C(CH_3)_3$ |
| 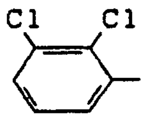 | $-(CH_2)_2-CH_3$ | $-C(CH_3)_3$ |
| 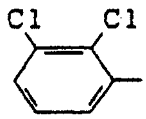 | $-CH(CH_3)_2$ | $-CH(C_2H_5)_2$ |
| 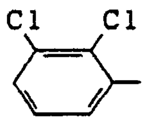 | 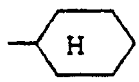 | $-CH(C_4H_9)_2$ |
| 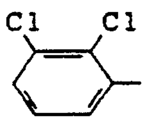 | $-CH_2CH_2-CN$ | $-C(CH_3)_3$ |
| 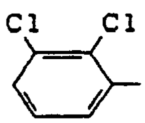 | $-CH_3$ | 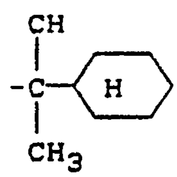 |
| 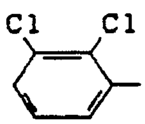 | $-CH_2CH{=}CH_2$ | $-C(CH_3)_3$ |
| 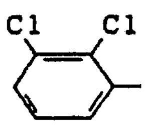 | 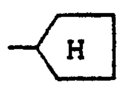 | $-C(CH_3)_3$ |
| 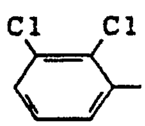 | 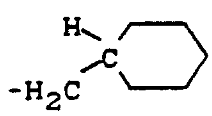 | $-CH(C_2H_5)_2$ |

Tabelle 1: - Fortsetzung

| Ar | $R^1$ | $R^2$ |
|---|---|---|
| 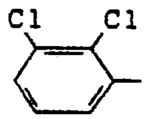 | $-sec-C_4H_9$ | 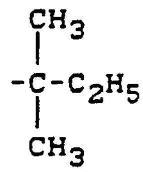 |
| 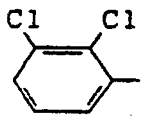 | $-iso-C_4H_9$ | $-C(CH_3)_3$ |
| 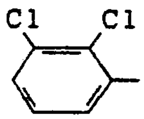 | 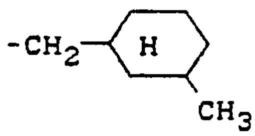 | $-C(CH_3)_3$ |
| 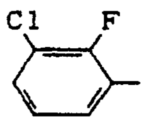 | $-C_2H_5$ | $-C(CH_3)_3$ |
| 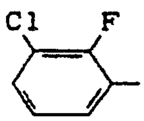 | $-(CH_2)_2-CH_3$ | $-C(CH_3)_3$ |
| 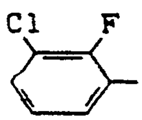 | $-CH(CH_3)_2$ | $-CH(C_2H_5)_2$ |
| 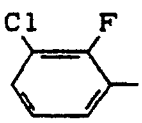 | 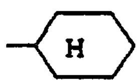 | $-CH(C_4H_9)_2$ |
| 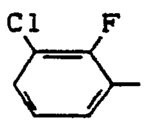 | $-CH_2CH_2-CN$ | $-C(CH_3)_3$ |

Tabelle 1: - Fortsetzung

| Ar | $R^1$ | $R^2$ |
|---|---|---|
| 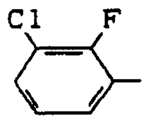 | $-CH_3$ | 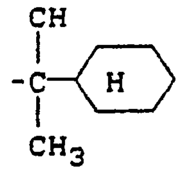 |
| 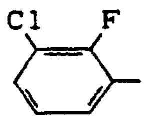 | $-CH_2CH{=}CH_2$ | $-C(CH_3)_3$ |
| 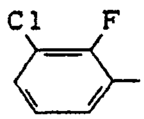 | 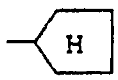 | $-C(CH_3)_3$ |
| 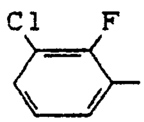 | 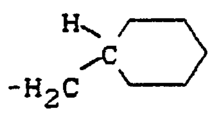 | $-CH(C_2H_5)_2$ |
| 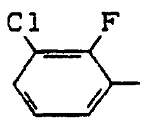 | -sec-$C_4H_9$ | 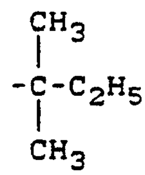 |
| 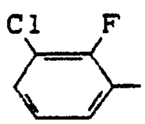 | -iso-$C_4H_9$ | $-C(CH_3)_3$ |
| 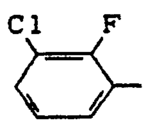 | 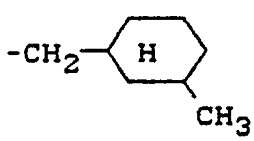 | $-C(CH_3)_3$ |
| 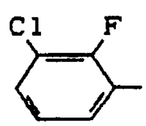 | 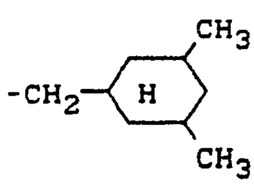 | $-C(CH_3)_3$ |

Tabelle 1: - Fortsetzung

| Ar | $R^1$ | $R^2$ |
|---|---|---|
| 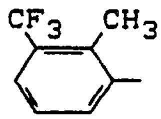 | $-C_2H_5$ | $-C(CH_3)_3$ |
| 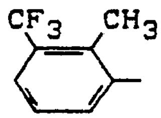 | $-(CH_2)_2-CH_3$ | $-C(CH_3)_3$ |
| 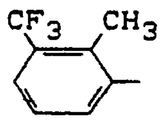 | $-CH(CH_3)_2$ | $-CH(C_2H_5)_2$ |
| 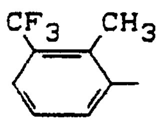 | 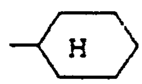 | $-CH(C_4H_9)_2$ |
| 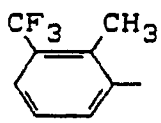 | $-CH_2CH_2-CN$ | $-C(CH_3)_3$ |
| 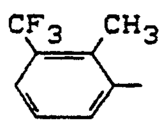 | $-CH_3$ | 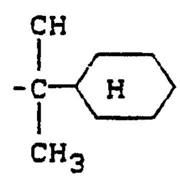 |
| 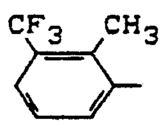 | $-CH_2CH=CH_2$ | $-C(CH_3)_3$ |
| 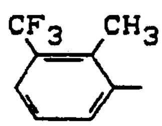 | 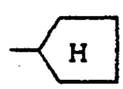 | $-C(CH_3)_3$ |
| 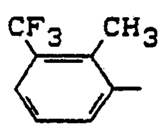 | 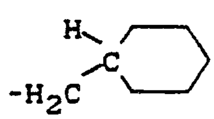 | $-CH(C_2H_5)_2$ |

Tabelle 1: - Fortsetzung

| Ar | $R^1$ | $R^2$ |
|---|---|---|
| 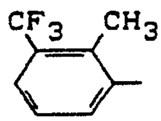 | $-sec-C_4H_9$ | 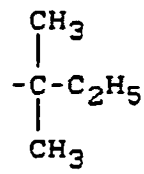 |
| 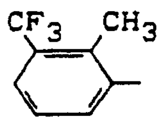 | $-iso-C_4H_9$ | $-C(CH_3)_3$ |
| 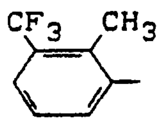 | 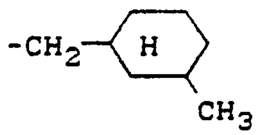 | $-C(CH_3)_3$ |
| 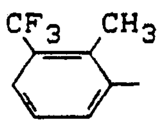 | 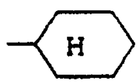 | 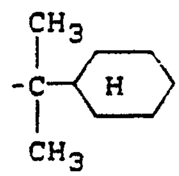 |
| 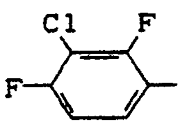 | $-C_2H_5$ | $-C(CH_3)_3$ |
| 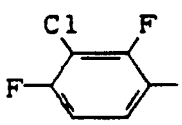 | $-(CH_2)_2-CH_3$ | $-C(CH_3)_3$ |
| 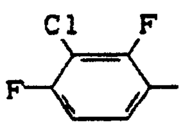 | $-CH(CH_3)_2$ | $-CH(C_2H_5)_2$ |
| 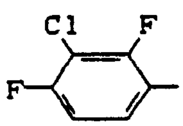 | 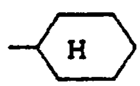 | $-CH(C_4H_9)_2$ |

Tabelle 1: - Fortsetzung

| Ar | $R^1$ | $R^2$ |
|---|---|---|
| 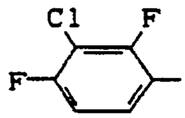 | $-CH_2CH_2-CN$ | $-C(CH_3)_3$ |
| 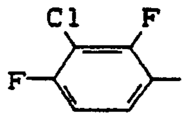 | $-CH_3$ | 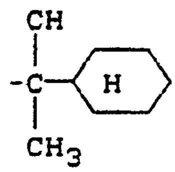 |
| 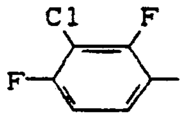 | $-CH_2CH=CH_2$ | $-C(CH_3)_3$ |
| 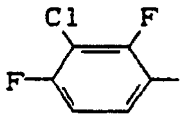 | 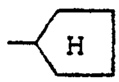 | $-C(CH_3)_3$ |
| 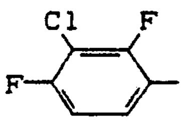 | 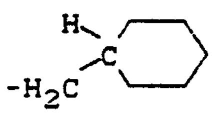 | $-CH(C_2H_5)_2$ |
| 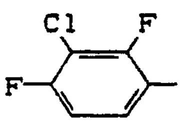 | $-sec-C_4H_9$ | 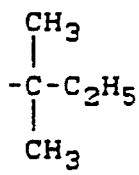 |
| 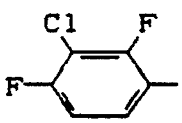 | $-iso-C_4H_9$ | $-C(CH_3)_3$ |
| 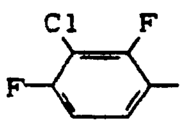 | 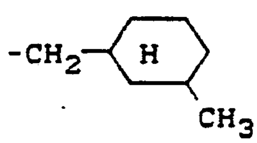 | $-C(CH_3)_3$ |
| 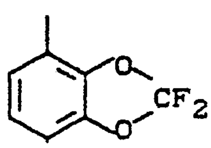 | $-C_2H_5$ | $-C(CH_3)_3$ |

Verwendet man beispielsweise 4-Cyano-3-(3-chlor-2-fluorphenyl)-pyrrol, Formaldehyd und N-Cyclohexyl-N-{[8-(1,1-dimethylpropyl)]-1,4-dioxaspiro[4.5]decan-2-yl-methyl}-amin als Ausgangsstoffe, so läßt sich der Reaktionsablauf des erfindungsgemäßen Verfahrens (a) durch das folgende Formelschema darstellen:

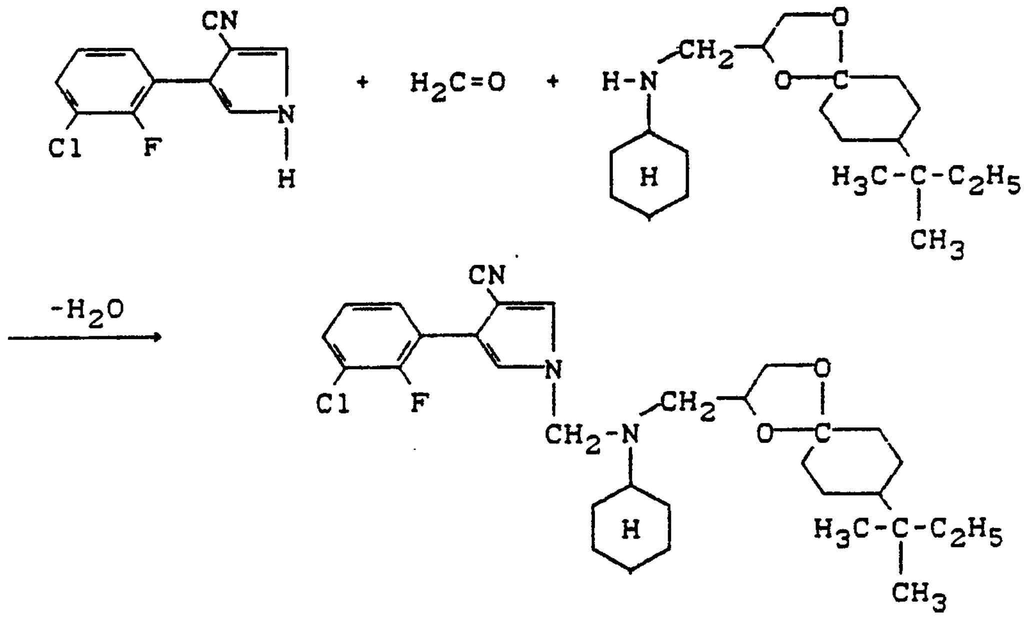


Verwendet man beispielsweise 1-Chlormethyl-4-cyano-3-(3-chlor-2-fluorphenyl)-pyrrol und N-Cyclohexyl-N-{[8-(1,1-dimethylpropyl)]-1,4-dioxaspiro[4.5]decan-2-yl-methyl}-amin als Ausgangsstoffe, so läßt sich der Reaktionsablauf des erfindungsgemäßen Verfahrens (b) durch das folgende Formelschema darstellen:

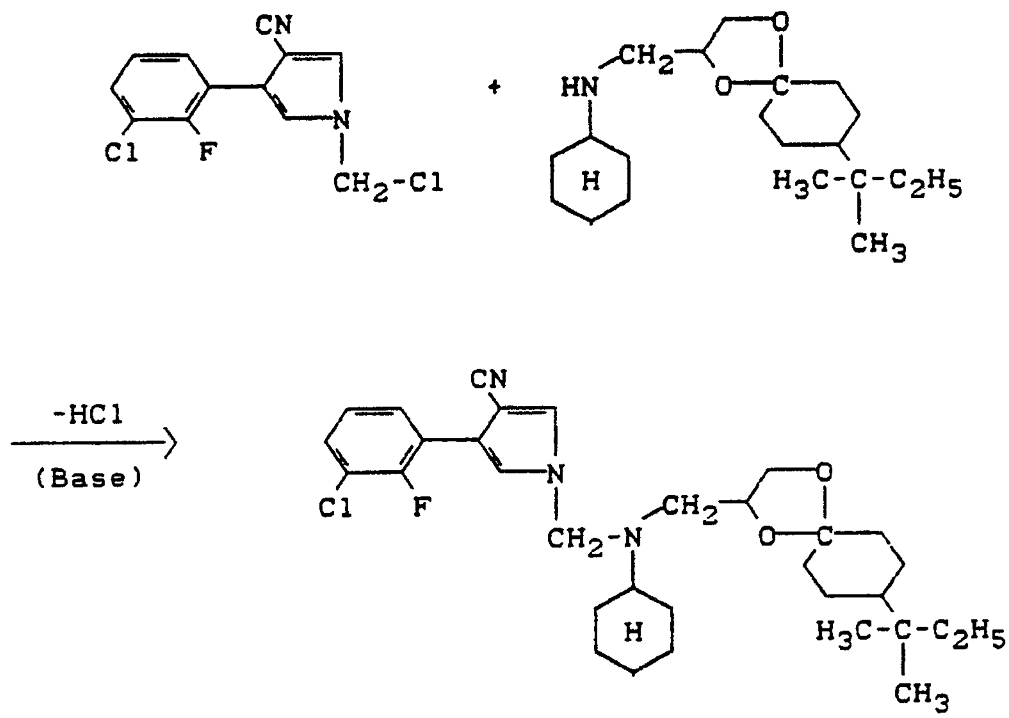


Die zur Durchführung des erfindungsgemäßen Verfahrens (a) als Ausgangsstoffe benötigten 3-Aryl-4-cyano-pyrrole sind durch die Formel (II) allgemein definiert. In dieser Formel (II) steht Ar vorzugsweise für diejenigen Reste, die bereits im Zusammenhang mit der Beschreibung der erfindungsgemäßen Stoffe der Formel (I) für diesen Substituenten genannt wurden.

Die 3-Aryl-4-cyano-pyrrole der Formel (II) sind bekannt (vgl. z.B. EP 174 910, EP 182 738 oder EP 133 247).

Die zur Durchführung des erfindungsgemäßen Verfahrens (b) als Ausgangsstoffe benötigten 3-Aryl-4-cyano-pyrrole sind durch die Formel (IV) allgemein definiert. In dieser Formel (IV) steht Ar vorzugsweise für diejenigen Reste, die bereits im Zusammenhang mit der Beschreibung der erfindungsgemäßen Stoffe der

Formel (I) für diesen Substituenten genannt wurden.

E steht vorzugsweise für Hydroxy oder Halogen, insbesondere für Chlor.

Die 3-Aryl-4-cyano-pyrrole der Formel (IV) sind ebenfalls bekannt (vgl. z.B. EP 133 247).

Die zur Durchführung der erfindungsgemäßen Verfahren (a) und (b) weiterhin als Ausgangsstoffe benötigten Amine sind durch die Formel (III) allgemein definiert. In dieser Formel (III) stehen $R^1$ und $R^2$ vorzugsweise für diejenigen Reste, die bereits im Zusammenhang mit der Beschreibung der erfindungsgemäßen Stoffe der Formel (I) für diese Substituenten genannt wurden.

Die Amine der Formel (III) sind allgemein bekannte Verbindungen der organischen Chemie oder erhältlich in Analogie zu bekannten Verfahren (vgl. z.B. GB 10 31 916 vom 2.6.1966; Org. Magnet. Res. 7, 488 - 495 [1975]; Kogyo Kagaku Zasshi 63, 1593 - 1597 [1960] bzw. CA 60: 10 542 f).

Als Verdünnungsmittel zur Durchführung des erfindungsgemäßen Verfahrens (a) kommen inerte organische Lösungsmittel oder wässrige Systeme infrage. Vorzugsweise verwendet man protische Lösungsmittel, beispielsweise Alkohole, wie Methanol, Ethanol oder Propanol oder Carbonsäuren, wie Ameisensäure, Essigsäure oder Propionsäure oder deren Gemische mit Wasser. Es ist auch möglich, das erfindungsgemäße Verfahren (a) in aprotischen Lösungsmitteln duchzuführen. Hierzu gehören insbesondere aliphatische oder aromatische, gegebenenfalls halogenierte Kohlenwasserstoffe, wie beispielweise Benzin, Benzol, Toluol, Xylol, Chlorbenzol, Petrolether, Hexan, Cyclohexan, Dichlormethan, Chloroform, Tetrachlorkohlenstoff, Ether, wie Diethylether, Dioxan, Tetrahydrofuran oder Ethylenglykoldimethyl-oder -diethylether, Ketone, wie Aceton oder Butanon, Nitrile, wie Acetonitril oder Propionitril, Amide, wie Dimethylformamid, Dimethylacetamid, N-Methylformanilid, N-Methylpyrrolidon oder Hexamethylphosphorsäuretriamid, Ester, wie Essigsäureethylester oder Sulfoxide, wie Dimethylsulfoxid.

Das erfindungsgemäße Verfahren (a) wird gegebenenfalls in Gegenwart eines geeigneten Reaktionshilfmittels durchgeführt. Hierzu eignen sich entweder katalytische bis äquimolare Mengen einer organischen oder anorganischen Säure oder entsprechende Mengen einer geeigneten Base.

Als saure Reaktionshilfsmittel kommen insbesondere anorganische Mineralsäuren, wie Phosphorsäure, Schwefelsäure, Salpetersäure, Chlorwasserstoffsäure oder Bromwasserstoffsäure oder organische Säuren, wie Ameisensäure, Essigsäure, Propionsäure, Methansulfonsäure, Benzolsulfonsäure oder Toluolsulfonsäure infrage.

Als basische Reaktionshilfsmittel kommen alle üblichen anorganischen oder organischen Basen in Frage. Hierzu gehören beispielsweise Alkalimetallhydroxide, wie Natriumhydroxid oder Kaliumhydroxid, Alkalimetallcarbonate, wie Natriumcarbonat, Kaliumcarbonat oder Natriumhydrogencarbonat, sowie tertiäre Amine, wie Triethylamin, N,N-Dimethylanilin, Pyridin, N,N-Dimethylaminopyridin, Diazabicyclooctan (DABCO), Diazabicyclononen(DBN) oder Diazabicycloundecen(DBU).

Es ist jedoch auch möglich, das als Reaktionspartner verwendete Amin der Formel (III) in entsprechendem Überschuß gleichzeitig als Reaktionshilfsmittel zu verwenden.

Die Reaktionstemperaturen können bei der Durchführung des erfindungsgemäßen Verfahrens (a) in einem größeren Bereich variiert werden. Im allgemeinen arbeitet man bei Temperaturen zwischen 0 °C und 120 °C, vorzugsweise bei Temperaturen zwischen 20 °C und 90 °C.

Zur Durchführung des erfindungsgemäßen Verfahrens (a) setzt man pro Mol an 3-Aryl-4-cyano-pyrrol der Formel (II) im allgemeinen 1,0 bis 2,0 Mol, vorzugsweise 1,0 bis 1,5 Mol an Amin der Formel (III) und 1,0 bis 2,0 Mol, vorzugsweise 1,0 bis 1,5 Mol an Formaldehyd ein. Der Formaldehyd wird entweder in Form einer wässrigen Lösung, als Paraformaldehyd oder als 1,3,5-Trioxan eingesetzt. Vorzugsweise verwendet man eine wässrige Lösung.

Die Reaktionsdurchführung, Aufarbeitung und Isolierung der Reaktionsprodukte der Formel (I) erfolgt in Analogie zu bekannten Verfahren (vgl. EP 133 247).

Als Verdünnungsmittel zur Durchführung des erfindungsgemäßen Verfahrens (b) kommen inerte organische Lösungsmittel infrage.

Hierzu gehören insbesondere aliphatische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff, Ether, wie Diethylether, Dioxan, Tetrahydrofuran oder Ethylenglykoldimethyl-oder -diethylether, Ketone, wie Aceton oder Butanon, Nitrile, wie Acetonitril oder Propionitril, Amide, wie Dimethylformamid, Dimethylacetamid, N-Methylformanilid, N-Methylpyrrolidon oder Hexamethylphosphorsäuretriamid, Ester, wie Essigsäureethylester oder Sulfoxide, wie Dimethylsulfoxid.

Das erfindungsgemäße Verfahren (b) wird gegebenenfalls in Gegenwart eines geeigneten Säurebindemittels durchgeführt. Als solche kommen alle üblichen anorganischen oder organischen Basen in Frage. Hierzu gehören beispielsweise Alkalimetallhydroxide, wie Natriumhydroxid oder Kaliumhydroxid, Alkalimetallcarbonate, wie Natriumcarbonat, Kaliumcarbonat oder Natriumhydrogencarbonat, sowie tertiäre Amine, wie Triethylamin, N,N-Dimethylanilin, Pyridin, N,N-Dimethylaminopyridin, Diazabicyclooctan (DABCO), Diazabicyclononen (DBN) oder Diazabicycloundecen(DBU).

Es ist jedoch auch möglich, das als Reaktionspartner in Frage kommende Amin der Formel (III) in entsprechendem Überschuß gleichzeitig als Reaktionshilfsmittel einzusetzen.

Die Reaktionstemperaturen können bei der Durchführung des erfindungsgemäßen Verfahrens (b) in einem größeren Bereich variiert werden. Im allgemeinen arbeitet man bei Temperaturen zwischen 0 °C und 100 °C, vorzugsweise bei Temperaturen zwischen 20 °C und 60 °C.

Zur Durchführung des erfindungsgemäßen Verfahrens (b) setzt man pro Mol an 3-Aryl-4-cyano-pyrrol der Formel (IV) im allgemeinen 1,0 bis 2,0 Mol, vorzugsweise 1,0 bis 1,5 Mol an Amin der Formel (III) und gegebenenfalls 1,0 bis 2,0 Mol, vorzugsweise 1,0 bis 1,5 Mol an Säurebindemittel ein. Die Reaktionsdurchführung, Aufarbeitung und Isolierung der Reaktionsprodukte der Formel (I) erfolgt in Analogie zu bekannten Verfahren.

Die erfindungsgemäßen Wirkstoffe weisen eine starke Wirkung gegen Schädlinge auf und können zur Bekämpfung von unerwünschten Schadorganismen praktisch eingesetzt werden. Die Wirkstoffe sind z.B. für den Gebrauch als Pflanzenschutzmittel insbesondere als Fungizide geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Beispielhaft aber nicht begrenzend seien einige Erreger von pilzlichen Erkrankungen, die unter die oben aufgezählten Oberbegriffe fallen, genannt:

Pythium-Arten, wie beispielsweise Pythium ultimum; Phytophthora-Arten, wie beispielsweise Phytophthora infestans;
Pseudoperonospora-Arten, wie beispielsweise Pseudoperonospora humuli oder Pseudoperonospora cubensis;
Plasmopara-Arten, wie beispielsweise Plasmopara viticola;
Peronospora-Arten, wie beispielsweise Peronospora pisi oder P. brassicae;
Erysiphe-Arten, wie beispielsweise Erysiphe graminis; Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis; Pyrenophora-Arten, wie beispielsweise Pyrenophora teres oder P. graminea
(Konidienform: Drechslera, Syn: Helminthosporium);
Cochliobolus-Arten, wie beispielsweise Cochliobolus sativus
(Konidienform: Drechslera, Syn: Helminthosporium);
Uromyces-Arten, wie beispielsweise Uromyces appendiculatus;
Puccinia-Arten, wie beispielsweise Puccinia recondita;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides;
Sclerotinia-Arten, wie z.B. Sclerotinia sclerotiorum.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Dabei lassen sich die erfindungsgemäßen Wirkstoffe mit besonders gutem Erfolg protektiv zur Bekämpfung von Botrytis bei Bohnen, Sphaerotheca bei Gurken und Leptosphaeria bei Weizen einsetzen. Hervorzuheben ist außerdem die sehr gute Wirksamkeit bei der Bekämpfung von Fusarium-culmorum bei der Saatgutbehandlung von Weizen.

Darüber hinaus zeigen die erfindungsgemäßen Verbindungen auch eine gute fungizide Wirkung gegen Erysiphe graminis an Getreidearten und gegen Pellicularia an Reis sowie eine gute in vitro-Wirkung.

Die Wirkstoffe können in Abhängigkeit von ihren jeweiligen physikalischen und/oder chemischen Eigenschaften in übliche Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe,

Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen in Mischung mit anderen bekannten Wirkstoffen vorliegen wie Fungizide, Insektizide, Akarizide und Herbizide sowie in Mischungen mit Düngemitteln und Wachstumsregulatoren.

Die Wirkstoffe konnen als solche, in Form ihrer Formulierungen oder den daraus bereiteten Anwendungsformen wie gebrauchsfertige Lösungen, Suspensionen, Spritzpulver, Pasten, lösliche Pulver, Stäubemittel und Granulate angewendet werden. Die Anwendung erfolgt in üblicher Art und Weise, z.B. durch Gießen, Verspritzen, Versprühen, Verstreuen, Verstäuben, Verschäumen, Bestreichen usw.. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g benötigt.

Bei Behandlung des Bodens sind Wirkstoffkonzentrationen von 0,00001 bis 0,1 Gew.-%, vorzugsweise von 0,0001 bis 0,02 % am Wirkungsort erforderlich.

Die erfindungsgemäßen Wirkstoffe zeichnen sich außerdem durch breite und starke mikrobizide Wirkung aus sowie durch auffallend gute Wirksamkeit gegen Algen und Schleimorganismen. Die erfindungsgemäßen Substanzen eignen sich daher vorzüglich zum Schutz technischer Materialien.

Technische Materialien sind erfindungsgemäß nicht lebende Materialien, die für die Verwendung in der Technik zubereitet worden sind. Beispielsweise können technische Materialien, die durch erfindungsgemäße Wirkstoffe vor mikrobieller Veränderung oder Zerstörung geschützt werden sollen, Klebstoffe, Leime, Papier und Karton, Textilien, Leder, Holz, Anstrichmittel und Kunststoffartikel, Kühlschmierstoffe und andere

Materialien sein, die von Mikroorganismen befallen oder zersetzt werden können. Im Rahmen der zu schützenden Materialien seien auch Teile von Produktionsanlagen, beispielsweise Kühlwasserkreisläufe, genannt, die durch Vermehrung von Mikroorganismen beeinträchtigt werden können. Im Rahmen der vorliegenden Erfindung seien als technische Materialien vorzugsweise Klebstoffe, Leime, Papiere und Kartone, Leder, Holz, Anstrichmittel, Kühlschmiermittel und Kühlkreisläufe genannt.

Als Mikroorganismen, die einen Abbau oder eine Veränderung der technischen Materialien bewirken können, seien beispielsweise Bakterien, Pilze, Hefen, Algen und Schleimorganismen genannt. Vorzugsweise wirken die erfindungsgemäßen Wirkstoffe gegen Pilze, insbesondere Schimmelpilze, holzverfärbende und holzzerstörende Pilze (Basidiomyceten), sowie gegen Schleimorganismen und Algen.

Es seien beispielsweise Mikroorganismen der folgenden Gattungen genannt:
Alternaria, wie Alternaria tenuis,
Aspergillus, wie Aspergillus niger,
Chaetomium, wie Chaetomium globosum,
Coniophora, wie Coniophora puteana,
Lentinus, wie Lentinus tigrinus,
Penicillium, wie Penicillium glaucum,
Polyporus, wie Polyporus versicolor,
Aureobasidium, wie Aureobasidium pullulans,
Sclerophoma, wie Sclerophoma pityophila,
Trichoderma, wie Trichoderma viride,
Escherichia, wie Escherichia coli,
Pseudomonas, wie Pseudomonas aeruginosa,
Staphylococcus, wie Staphylococcus aureus.

Je nach Anwendungsgebiet kann ein erfindungsgemäßer Wirkstoff in die üblichen Formulierungen überführt werden, wie Lösungen, Emulsionen, Suspensionen, Pulver, Pasten und Granulate.

Diese können in an sich bekannter Weise hergestellt werden, z.B. durch Vermischen der Wirkstoffe mit einem Streckmittel, das aus flüssigem Lösungsmittel und/oder festen Trägerstoffen besteht, gegebenenfalls unter Verwendung von oberflächenaktiven Mitteln, wie Emulgatoren und/oder Dispergiermitteln, wobei gegebenenfalls im Falle der Benutzung von Wasser als Streckmittel organische Lösungsmittel wie Alkohole als Hilfsmittel verwendet werden können.

Flüssige Lösungsmittel für die Wirkstoffe können beispielsweise Wasser, Alkohole, wie niedere aliphatische Alkohole, vorzugsweise Ethanol oder Isopropanol, oder Benzylalkohol, Ketone, wie Aceton oder Methylethylketon, flüssige Kohlenwasserstoffe, wie Benzinfraktionen, halogenierte Kohlenwasserstoffe, wie 1,2-Dichlorethan, sein.

Mikrobizide Mittel enthalten die Wirkstoffe im allgemeinen in einer Menge von 1 bis 95 %, bevorzugt von 10 bis 75 %.

Die Anwendungskonzentrationen der erfindungsgemäßen Wirkstoffe richten sich nach der Art und dem Vorkommen der zu bekämpfenden Mikroorganismen, sowie nach der Zusammensetzung des zu schützenden Materials. Die optimale Einsatzmenge kann durch Testreihen ermittelt werden. Im allgemeinen liegen die Anwendungskonzentrationen im Bereich von 0,001 bis 5 Gew.-%, vorzugsweise von 0,05 bis 1,0 Gew.-%, bezogen auf das zu schützende Material.

Die erfindungsgemäßen Wirkstoffe können auch in Mischung mit anderen bekannten Wirkstoffen vorliegen. Beispielsweise seien die folgenden Wirkstoffe genannt: Benzylalkoholmono(poly)hemiformal und andere Formaldehyd abspaltende Verbindungen, Benzimidazolyl-methylcarbamate, Tetramethylthiuramdisulfid, Zinksalze von Dialkyldithiocarbamaten, 2,4,5,6-Tetrachlorisophthalonitril, Thiazolylbenzimidazol, Mercaptobenzthiazol, 2-Rhodanido-methylthiobenzthiazol, Organo-Zinnverbindungen, Methylenbisthiocyanat, N-Alkyl- und N-Aryliodpropargylcarbamate, Triazol- und Imidazolfungizide, Phenolderivate, wie 2-Phenylphenol, (2,2'-Dihydroxy-5,5'-dichlor)-di-phenylmethan und 3-Methyl-4-chlor-phenol.

Die Herstellung und die Verwendung der erfindungsgemäßen Wirkstoffe geht aus den nachfolgenden Beispielen hervor:

## Herstellungsbeispiele

### Beispiel 1

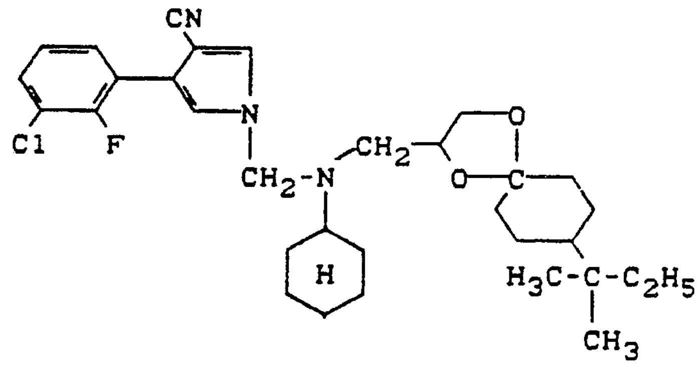

(Verfahren a)

Zu 2,0 g (9,064 mMol) 4-Cyano-3-(2-fluor-3-chlorphenyl)-pyrrol in 10 ml Ethanol gibt man 0,6 g (9,697 mMol) 37-prozentige wässrige Formaldehydlösung und 0,1 ml Eisessig. Danach gibt man tropfenweise unter Rühren 3,1 g (9,697 mMol) N-Cyclohexyl-[8-(1,1-dimethylpropyl)-1,4-dioxaspiro[4.5]decan]-2-methanamin zu und rührt nach beendeter Zugabe 20 Stunden bei Raumtemperatur. Zur Aufarbeitung gibt man 50 ml Ethylacetat zu, wäscht dreimal mit Wasser, trocknet über Natriumsulfat, entfernt das Lösungsmittel im Vakuum und kristallisiert den Rückstand aus Ether/Cyclohexan.
Man erhält 4-Cyano-3-(2-fluor-3-chlorphenyl)-1-(N-cyclohexyl-1,4-dioxaspiro[4.5]decan-8-(1,1-dimethylpropyl)-2-methanaminomethyl)-pyrrol in quantitativer Ausbeute als braunes Öl.

In analoger Weise zu Beispiel 1 und unter Berücksichtigung der Angaben in den Beschreibungen der erfindungsgemäßen Verfahren, werden die nachstehend in Tabelle 2 aufgeführten Endprodukte der Formel (I)

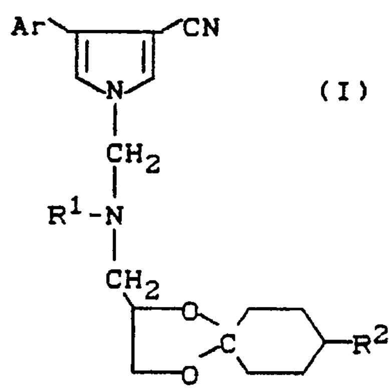

(I)

erhalten:

Tabelle 2:

| Bsp.-Nr. | Ar | $R^1$ | $R^2$ |
|---|---|---|---|
| 2 | 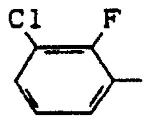 | 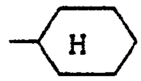 | 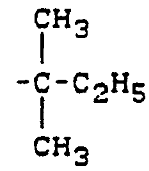 |
| 3 | 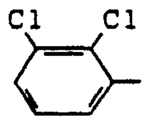 | 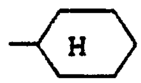 | 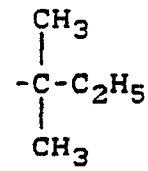 |
| 4 | 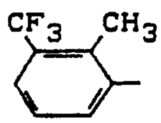 | $-C_4H_9$-n | 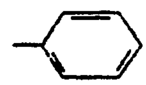 |

<u>Tabelle 2:</u> - Fortsetzung

| Bsp.-Nr. | Ar | $R^1$ | $R^2$ |
|---|---|---|---|
| 5 | 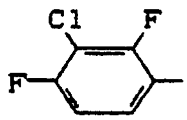 | $-CH_2CH_2-OCH_3$ | 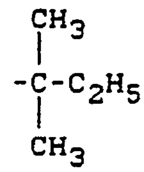 |
| 6 | 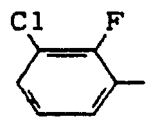 | $-CH_2CH_2-OCH_3$ | 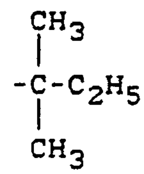 |
| 7 | 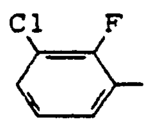 | $-(CH_2)_3-OCH_3$ | $-C(CH_3)_3$ |
| 8 | 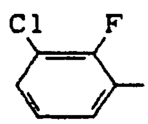 | $-C_4H_9-n$ | 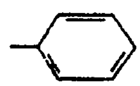 |
| 9 | 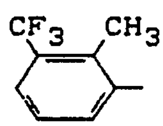 | $-C_5H_{11}-n$ | $-CH(C_2H_5)_2$ |
| 10 | 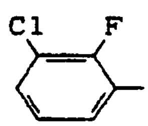 | $-C_5H_{11}-n$ | $-CH(C_2H_5)_2$ |
| 11 | 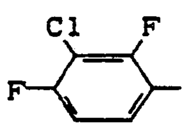 | $-C_5H_{11}-n$ | $-CH(C_2H_5)_2$ |
| 12 | 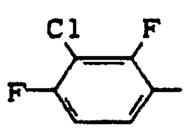 | $-(CH_2)_3-OCH_3$ | $-C(CH_3)_3$ |

Tabelle 2: - Fortsetzung

| Bsp.-Nr. | Ar | $R^1$ | $R^2$ |
|---|---|---|---|
| 13 | 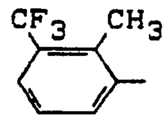 | $-(CH_2)_3-OCH_3$ | $-C(CH_3)_3$ |
| 14 | 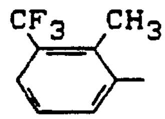 | $-(CH_2)_2-OCH_3$ | 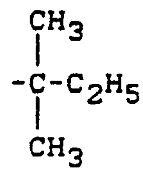 |
| 15 | 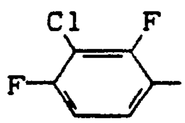 | 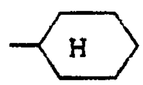 | 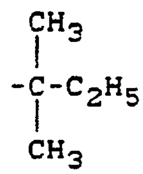 |
| 16 | 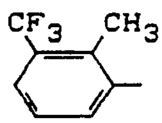 | 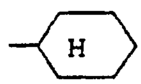 | 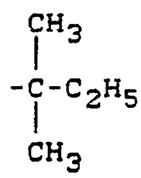 |
| 17 | 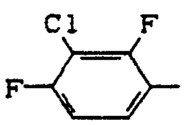 | $-C_4H_9-n$ | 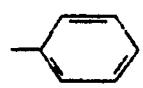 |

Die Verbindungen der Beispiele 1 bis 17 fallen in Form von zähen Ölen an. Die Identifizierung erfolgt über $^1$H-NMR.

Die $^1$H-NMR-Spektren wurden in Deuterochloroform ($CDCl_3$) mit Tetramethylsilan (TMS) als inneren Standard aufgenommen.

Die physikalischen Daten für die präparativen Beispiele siehe die folgende Tabelle 3.

Tabelle 3: physikalische Konstanten

| Bsp.-Nr. | Struktur | $^{1}$H-NMR-Daten in $CDCl_3$ (300 MHz) |
|---|---|---|
| | | δ |
| 1) | 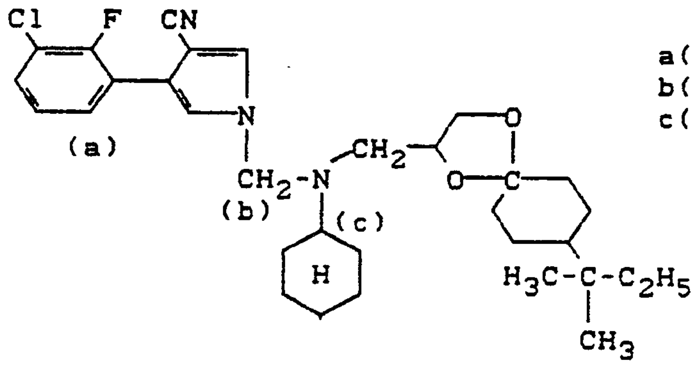 | a(CH) = 7,73 M<br>b($CH_2$)= 4,87 M<br>c(CH) = 2,6 M |
| 2) | 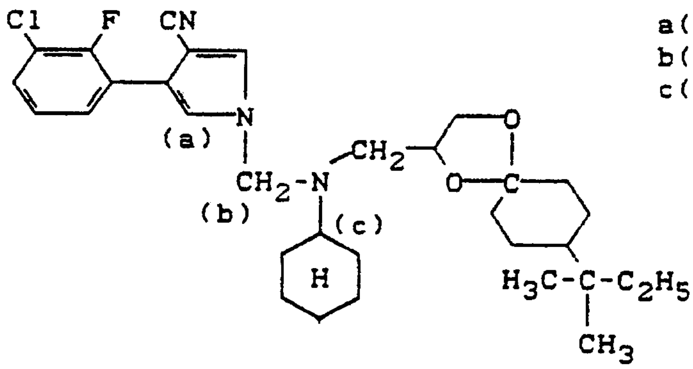 | a(CH) = 7,2 S<br>b($CH_2$)= 4,88 M<br>c(CH) = 2,59 M |
| 3) | 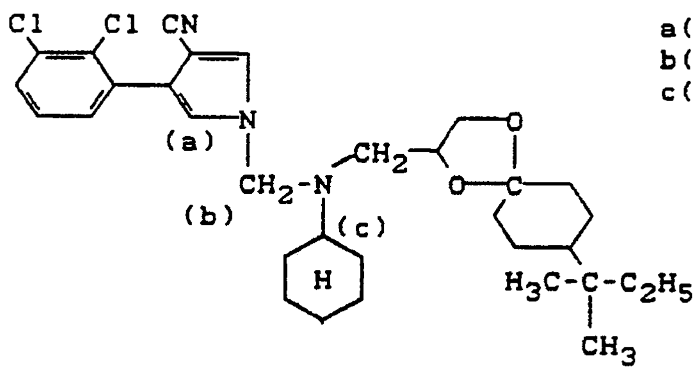 | a(CH) = 7,08 S<br>b($CH_2$)= 4,90 M<br>c(CH) = 2,58 M |

Tabelle 3: physikalische Konstanten - Fortsetzung

| Bsp.-Nr. | Struktur | $^1$H-NMR-Daten in $CDCl_3$ (300 MHz) δ |
|---|---|---|
| 4) | 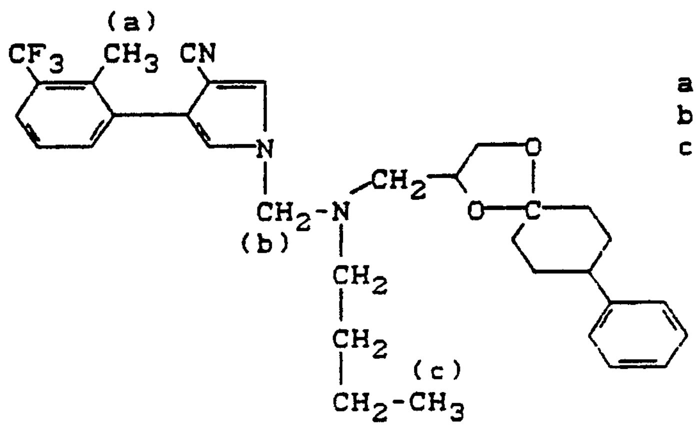 | a($CH_3$) = 2,40 S<br>b($CH_2$) = 4,89 M<br>c($CH_3$) = 0,90 M(T) |
| 5) | 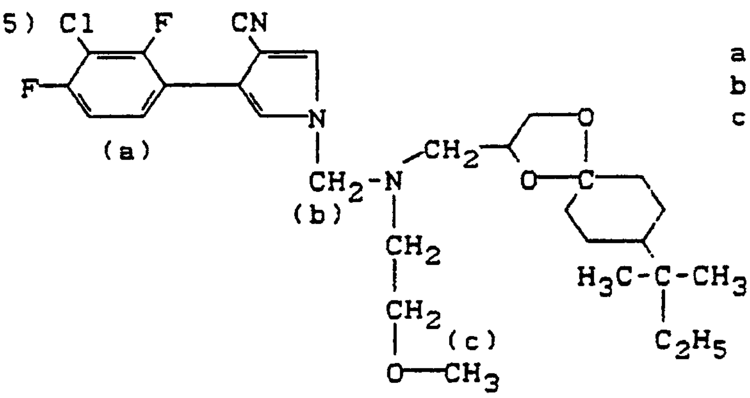 | a(CH) = 7,65 M<br>b($CH_2$) = 4,98 M<br>c($CH_3$) = 3,35 S |
| 6) | 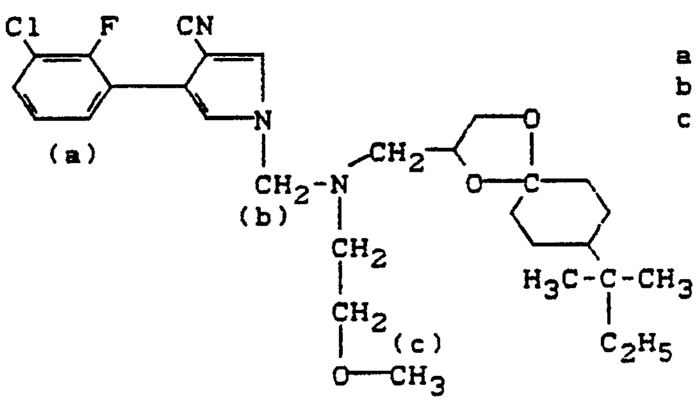 | a(CH) = 7,75 M<br>b($CH_2$) = 5,01 M<br>c($CH_3$) = 3,35 S |

Tabelle 3: physikalische Konstanten - Fortsetzung

| Bsp.-Nr. | Struktur | $^{1}$H-NMR-Daten in $CDCl_3$ (300 MHz) δ |
|---|---|---|
| 7) | 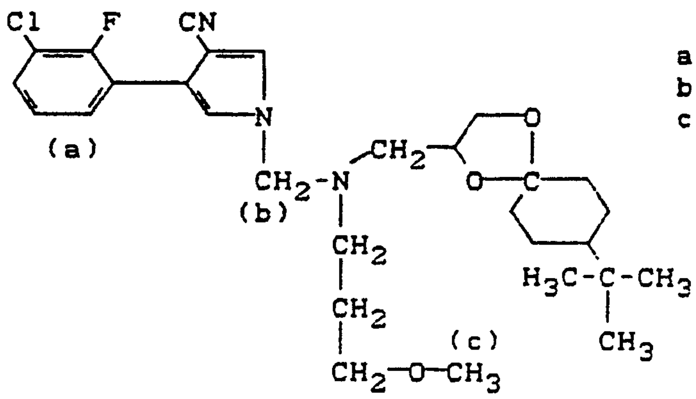 | a(CH) = 7,75 M<br>b($CH_2$)= 4,92 M<br>c($CH_3$)= 3,30 S |
| 8) | 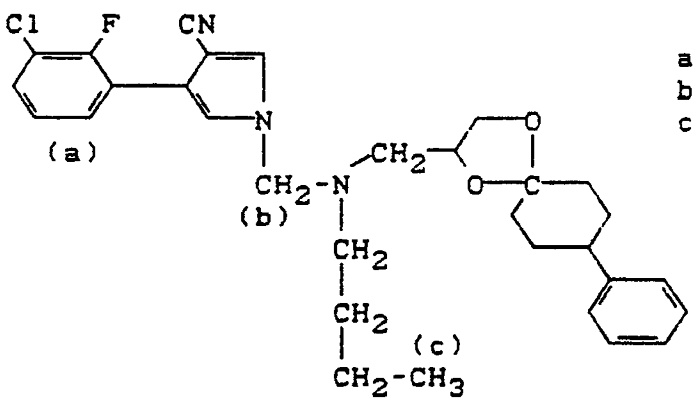 | a(CH) = 7,75 M<br>b($CH_2$)= 4,93 M<br>c($CH_3$)= 0,86 M |
| 9) | 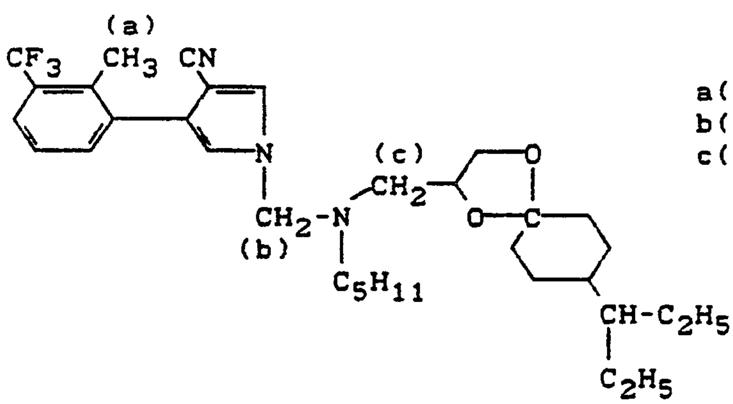 | a($CH_3$)= 2,41 S<br>b($CH_2$)= 4,88 M<br>c($CH_2$)= 2,63 M |

Tabelle 3: physikalische Konstanten - Fortsetzung

| Bsp.-Nr. | Struktur | $^{1}$H-NMR-Daten in $CDCl_3$ (300 MHz) δ |
| --- | --- | --- |
| 10) | 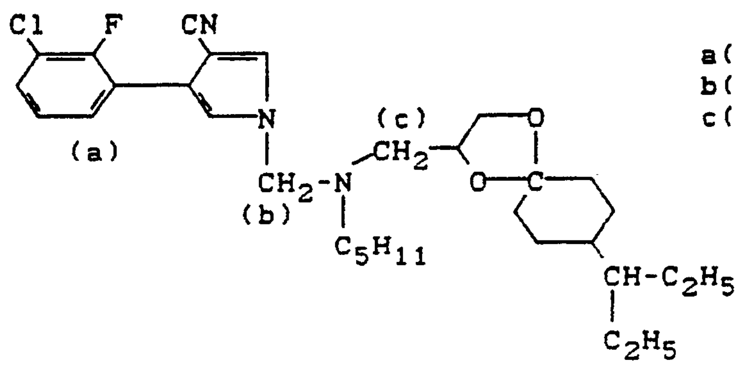 | a(CH) = 7,72 M<br>b($CH_2$)= 4,91 M<br>c($CH_2$)= 2,59 M |
| 11) | 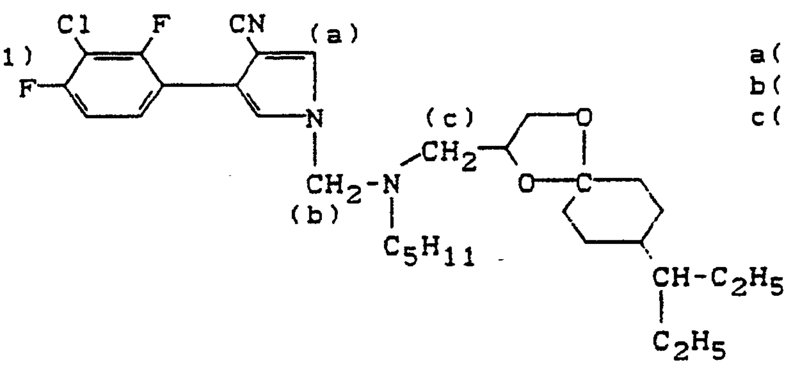 | a(CH) = 7,38 S<br>b($CH_2$)= 4,87 M<br>c($CH_2$)= 2,65 M |
| 12) | 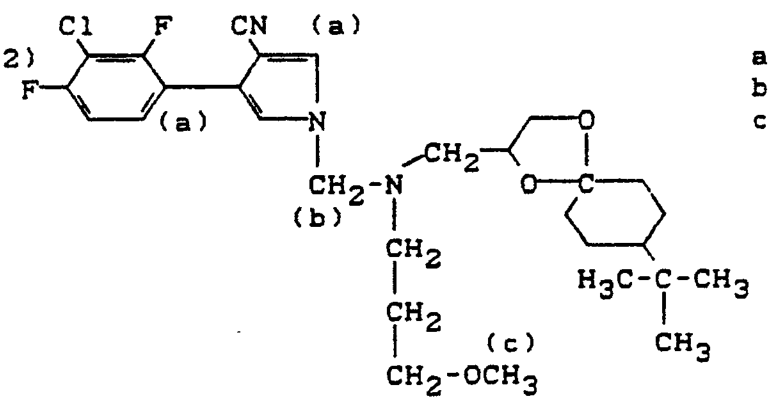 | a(CH) = 7,4 S<br>b($CH_2$)= 4,91 M<br>c($CH_3$)= 3,32 S |

Tabelle 3: physikalische Konstanten - Fortsetzung

| Bsp.-Nr. | Struktur | $^1$H-NMR-Daten in $CDCl_3$ (300 MHz) δ |
|---|---|---|
| 13) | 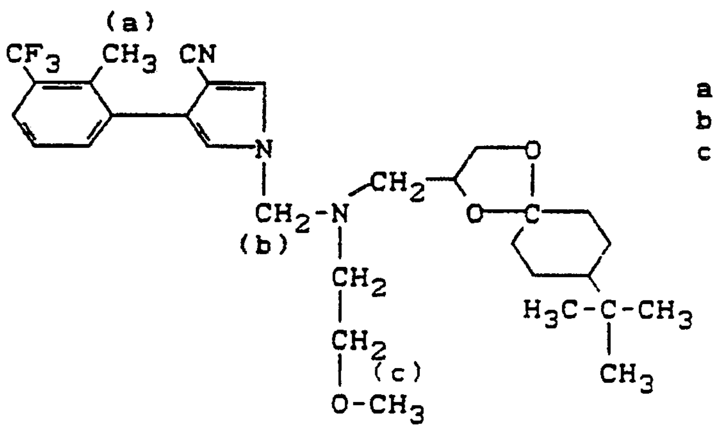 | a($CH_3$)= 2,45 S<br>b($CH_2$)= 4,88 M<br>c($CH_3$)= 3,3 S |
| 14) | 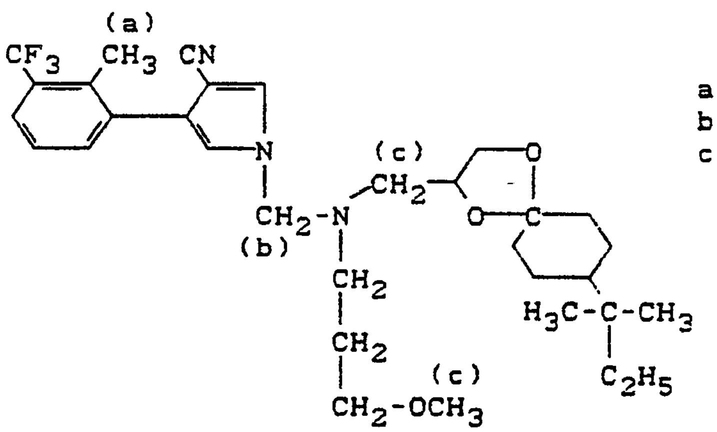 | a($CH_3$)= 2,42 S<br>b($CH_2$)= 4,98 M<br>c($CH_3$)= 3,35 S |
| 15) | 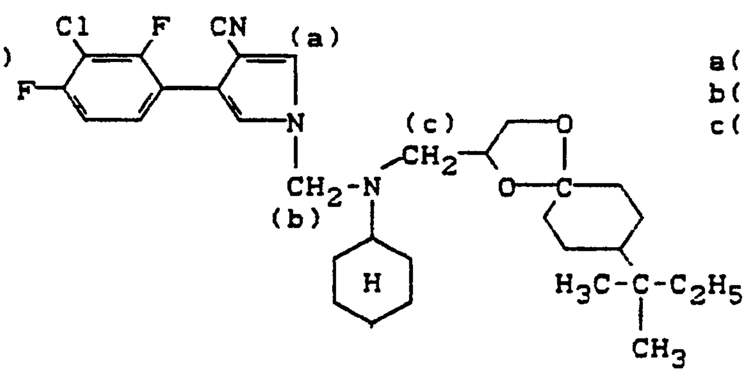 | a(CH) = 7,43 S<br>b($CH_2$)= 4,88 M<br>c($CH_2$)= 2,8 M |

Tabelle 3: physikalische Konstanten - Fortsetzung

| Bsp.-Nr. | Struktur | $^1$H-NMR-Daten in $CDCl_3$ (300 MHz) δ |
|---|---|---|
| 16) | 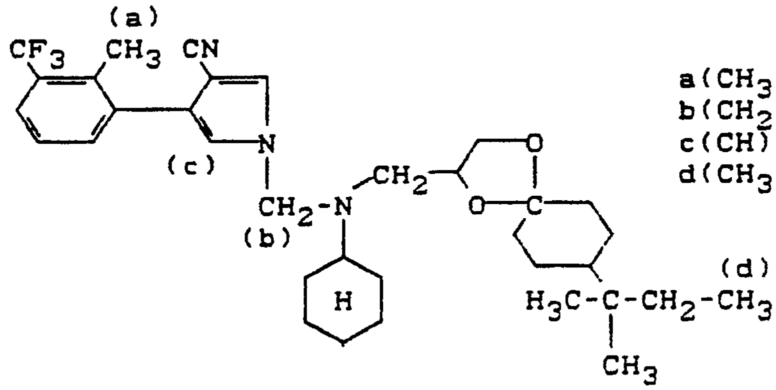 | a($CH_3$)= 2,42 S<br>b($CH_2$)= 4,88 M<br>c(CH) = 6,78 S<br>d($CH_3$)= 0,8 M(T) |
| 17) | 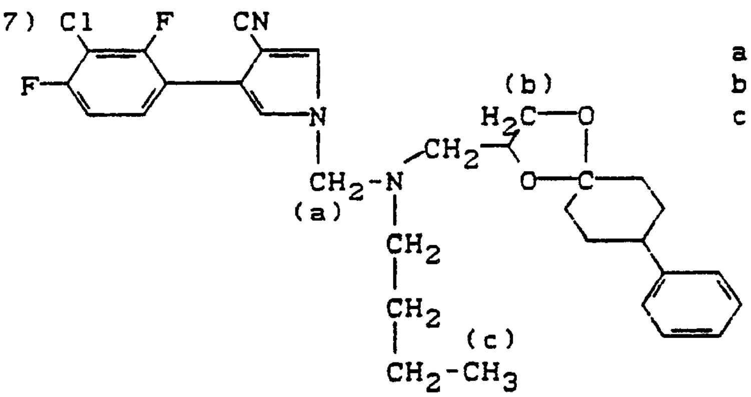 | a($CH_2$)= 4,9 M<br>b($CH_2$)= 3,68 M<br>c($CH_3$)= 0,9 M(T) |

M = Multiplett

S = Singulett

(T)= Triplettcharakter

Anwendungsbeispiele

In den folgenden Anwendungsbeispielen wurden die nachstehend aufgeführten Verbindungen als Vergleichssubstanzen eingesetzt:

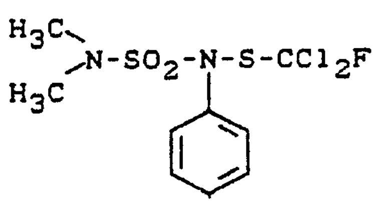

(A)

N,N-Dimethyl-N' -( fluordichlormethylsulfenyl)-sulfamid

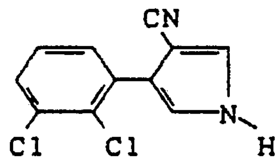

(B)

4-Cyano-3-(2,3-dichlorphenyl)-pyrrol
(bekannt aus EP 174 910)

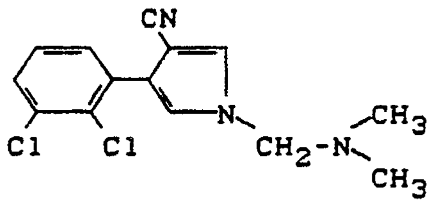

(C)

4-Cyano-3-(2,3-dichlorphenyl)-1-(N,N-dimethylaminomethyl)-pyrrol
(bekannt aus EP 133 247 und EP 182 738).

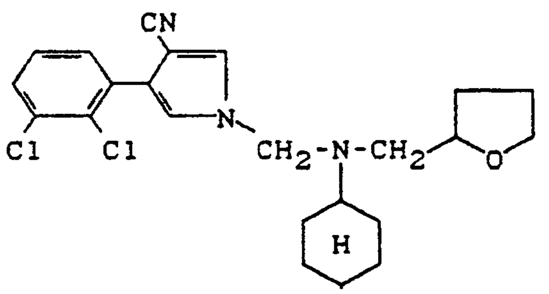

(D)

4-Cyano-3-(2,3-dichlor-phenyl)-1-(N-cyclohexyl-N-tetrahydrofurfurylaminomethyl)-pyrrol
(bekannt aus EP 281 731)

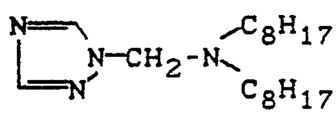

(E)

1,2,4-Triazol-1-yl-methyl-di-n-octylamin
(bekannt aus EP 106 243).

Beispiel A

Botrytis-Test (Bohne)/protektiv

Lösungsmittel: 4.7 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden auf jedes Blatt 2 kleine mit Botrytis cinerea bewachsene Agarstückchen aufgelegt. Die inokulierten Pflanzen werden in einer abgedunkelten, feuchten

Kammer bei 20 °C aufgestellt. 3 Tage nach der Inokulation wird die Größe der Befallsflecken auf den Blättern ausgewertet.

Eine deutliche Überlegenheit in der Wirksamkeit gegenüber dem Stand der Technik zeigt in diesem Test z.B. die Verbindung gemäß Herstellungsbeispiel 1.

Beispiel B

Sphaerotheca-Test (Gurke) / protektiv

Lösungsmittel: 4,7 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit Konidien des Pilzes Sphaerotheca fuliginea bestäubt.

Die Pflanzen werden anschließend bei 23 bis 24 °C und bei einer relativen Luftfeuchtigkeit von ca. 75 % im Gewächshaus aufgestellt.

10 Tage nach der Inokulation erfolgt die Auswertung.

Eine deutliche Überlegenheit in der Wirksamkeit gegenüber dem Stand der Technik zeigen in diesem Test z.B. die Verbindungen gemäß den Herstellungsbeispielen: 1, 2 und 3.

Beispiel C

Leptosphaeria nodorum-Test (Weizen)/protektiv

Lösungsmittel: 100 Gewichtsteile Dimethylformamid
Emulgator: 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit einer Konidiensuspension von Leptosphaeria nodorum besprüht. Die Pflanzen verbleiben 48 Stunden bei 20 °C und 100 % relativer Luftfeuchtigkeit in einer Inkubationskabine.

Die Pflanzen werden in einem Gewächshaus bei einer Temperatur von ca. 15 °C und einer relativen Luftfeuchtigkeit von ca. 80 % aufgestellt.

10 Tage nach der Inokulation erfolgt die Auswertung.

Eine deutliche Überlegenheit in der Wirksamkeit gegenüber dem Stand der Technik zeigt bei diesem Test z.B. die Verbindung gemäß dem Herstellungsbeispiel 1.

Beispiel D

Fusarium culmorum-Test (Weizen) / Saatgutbehandlung

Die Anwendung der Wirkstoffe erfolgt als Trockenbeizmittel. Sie werden zubereitet durch Abstrecken des jeweiligen Wirkstoffes mit Gesteinsmehl zu einer feinpulvrigen Mischung, die eine gleichmäßige Verteilung auf der Saatgutoberfläche gewährleistet.

Zur Beizung schüttelt man das infizierte Saatgut 3 Minuten lang mit dem Beizmittel in einer verschlossenen Glasflasche.

Den Weizen sät man mit 2 x 100 Korn 1 cm tief in eine Standarderde und kultiviert ihn im Gewächshaus bei einer Temperatur von ca. 18 °C in Saatkästen, die täglich 15 Stunden dem Licht ausgesetzt werden.

Ca. 3 Wochen nach der Aussaat erfolgt die Auswertung der Pflanzen auf Symptome.

Eine deutliche Überlegenheit in der Wirksamkeit gegenüber dem Stand der Technik zeigt bei diesem Test z.B. die Verbindung gemäß dem Herstellungsbeispiel 1.

Beispiel E

Minimale Hemmkonzentration

Zum Nachweis der Wirksamkeit gegen Pilze werden die minimalen Hemm-Konzentrationen (MHK) von erfindungsgemäßen Wirkstoffen bestimmt:

Ein Agar, der aus Bierwürze und Pepton hergestellt wird, wird mit erfindungsgegmäßen Wirkstoffen in Konzentrationen von 0,1 mg/l bis 5000 mg/l versetzt. Nach Erstarren des Agars erfolgt Kontamination mit Reinkulturen der in der Tabelle aufgeführten Testorganismen. Nach 2-wöchiger Lagerung bei 28° C und 60 bis 70 % realtiver Luftfeuchtigkeit wird die MHK bestimmt. MHK ist die niedrigste Konzentration an Wirkstoff, bei der keinerlei Bewuchs durch die verwendete Mikrobenart erfolgt. Bei vielen der erfindungsgemäßen Verbindungen liegt sie zwischen 20-50 mg/l.

**Ansprüche**

1. Substituierte 1-Aminomethyl-3-aryl-4-cyano-pyrrole der allgemeinen Formel (I),

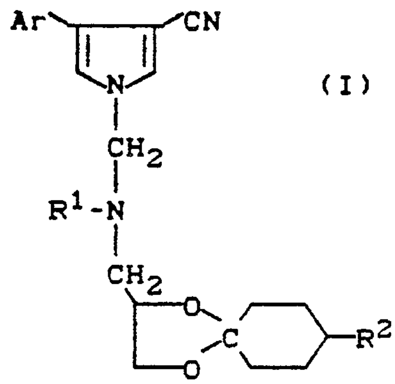

(I)

in welcher

Ar für gegebenenfalls substituiertes Phenyl steht,

$R^1$ für gegebenenfalls substituiertes Alkyl, für Alkenyl, Alkinyl oder Cycloalkyl oder für jeweils gegebenenfalls substituiertes Aralkyl oder Aryl steht und

$R^2$ für Alkyl oder für jeweils gegebenenfalls substituiertes Cycloalkylalkyl, Aralkyl, Cycloalkyl oder Aryl steht

und deren Säureadditionssalze, Isomere und Isomerengemische.

2. Substituierte 1-Aminomethyl-3-aryl-4-cyano-pyrrole gemäß Anspruch 1 der Formel (I), bei welchen

Ar für gegebenenfalls einfach bis mehrfach, gleich oder verschieden substituiertes Phenyl steht, wobei als Substituenten infrage kommen: Halogen, jeweils geradkettiges oder verzweigtes Alkyl, Alkoxy, Alkylthio, Halogenalkyl, Halogenalkoxy oder Halogenalkylthio mit jeweils 1 bis 4 Kohlenstoffatomen und gegebenenfalls 1 bis 9 gleichen oder verschiedenen Halogenatomen oder Difluormethylendioxy,

$R^1$ für gegebenenfalls substituiertes, geradkettiges oder verzweigtes Alkyl mit 1 bis 6 Kohlenstoffatomen steht, wobei als Substituenten infrage kommen: Cyano, Cycloalkyl mit 3 bis 7 Kohlenstoffatomen, jeweils geradkettiges oder verzweigtes Alkoxy, Alkylcarbonyl, Alkoxycarbonyl, Alkylaminocarbonyl, Dialkylaminocarbonyl, Alkylsulfinyl, Alkylsulfonyl oder Dialkylamino mit jeweils 1 bis 6 Kohlenstoffatomen in den einzelnen Alkylteilen; außerdem für jeweils geradkettiges oder verzweigtes Alkenyl oder Alkinyl mit jeweils 3 bis 6 Kohlenstoffatomen, für Cycloalkyl mit 3 bis 7 Kohlenstoffatomen oder für jeweils gegebenenfalls einfach bis mehrfach, gleich oder verschieden im Phenylteil substituiertes Phenyl oder Benzyl steht, wobei als Substituenten jeweils infrage kommen: Halogen, Cyano, jeweils geradkettiges oder verzweigtes Alkyl, Alkoxy, Halogenalkyl, Halogenalkoxy, Alkylcarbonyl oder Alkoxycarbonyl mit jeweils 1 bis 6 Kohlenstoffatomen in den einzelnen Alkylteilen und im Fall des Halogenalkyl- oder Halogenalkoxy-Restes mit jeweils 1 bis 9 gleichen oder verschiedenen Halogenatomen und

$R^2$ für geradkettiges oder verzweigtes Alkyl mit 1 bis 14 Kohlenstoffatomen, für jeweils gegebenenfalls einfach bis mehrfach, gleich oder verschieden durch Halogen und/oder geradkettiges oder verzweigtes Alkyl mit 1 bis 8 Kohlenstoffatomen substituiertes Cycloalkylalkyl oder Cycloalkyl mit jeweils 3 bis 7 Kohlenstoffatomen im Cycloalkylteil und gegebenenfalls 1 bis 6 Kohlenstoffatomen im geradkettigen oder verzweigten Alkylteil oder für jeweils gegebenenfalls einfach bis mehrfach, gleich oder verschieden durch Halogen und/oder geradkettiges oder verzweigtes Alkyl mit 1 bis 8 Kohlenstoffatomen substituiertes Aralkyl oder Aryl mit jeweils 6 bis 10 Kohlenstoffatomen im Arylteil und gegebenenfalls 1 bis 6 Kohlenstoffastomen im geradkettigen oder verzweigten Alkylteil steht.

**3.** Substituierte 1-Aminomethyl-3-aryl-4-cyano-pyrrole der Formel (I) gemäß Anspruch 1, bei welchen

Ar für gegebenenfalls ein- bis dreifach, gleich oder verschieden substituiertes Phenyl steht, wobei als Substituenten infrage kommen: Fluor, Chlor, Brom, Methyl, Ethyl, n- oder i-Propyl, n-, i-, s- oder t-Butyl, Methoxy, Ethoxy, Methylthio, Trifluormethyl, Trifluormethoxy, Trifluormethylthio oder Difluormethylendioxy,

$R^1$ für Methyl, Ethyl, n- oder i-Propyl, n-, i- oder s-Butyl, Pentyl oder Hexyl, für Cyclohexylmethyl oder durch 1 - 3 Methylgruppen substituiertes Cyclohexylmethyl steht, außerdem für Allyl, n-oder i-Butenyl, Propargyl, n- oder i-Butinyl, für Cyclopentyl, Cyclohexyl oder Cyclopropyl steht, wobei diese durch 1 - 3 Methylgruppen substituiert sein können oder für jeweils gegebenenfalls ein- bis dreifach, gleich oder verschieden im Phenylteil substituiertes Benzyl oder Phenyl steht, wobei als Substituenten infrage kommen: Fluor, Chlor, Brom, Cyano, Methyl, Ethyl, n- oder i-Propyl, n-, i- oder s-Butyl, Methoxy, Ethoxy, Trifluormethyl, Trifluormethoxy, Acetyl, Methoxycarbonyl oder Ethoxycarbonyl und

$R^2$ für geradkettiges oder verzweigtes Alkyl mit 1 bis 12 Kohlenstoffatomen, für gegebenenfalls ein- bis dreifach, gleich oder verschieden durch Methyl, Ethyl, n- oder i-Propyl, n-, i-, s- oder t-Butyl substituiertes Cyclohexyl, für im Cyclohexylteil gegebenenfalls ein- bis dreifach, gleich oder verschieden durch Methyl, Ethyl, n- oder i-Propyl, n-, i-, s- oder t-Butyl substituiertes Cyclohexylalkyl mit 1 bis 4 Kohlenstoffatomen im geradkettigen oder verzweigten Alkylteil, für gegebenenfalls ein- bis dreifach, gleich oder verschieden durch Methyl, Ethyl, n- oder i-Propyl, n-, i-, s-oder t-Butyl substituiertes Phenyl oder für im Phenylteil gegebenenfalls ein- bis dreifach, gleich oder verschieden durch Methyl, Ethyl, n- oder i-Propyl, n-, i-, s-oder t-Butyl substituiertes Phenylalkyl mit 1 bis 4 Kohlenstoffatomen im geradkettigen oder verzweigten Alkylteil steht.

**4.** Substituierte 1-Aminomethyl-3-aryl-4-cyano-pyrrole der Formel (I) gemäß Anspruch 1, bei welchen

Ar für dreifach, gleich oder verschieden durch Chlor, Fluor, Methyl, Trifluormethyl oder Trifluormethoxy substituiertes Phenyl steht,

$R^1$ für Ethyl, n- oder i-Propyl, n-, i- oder s-Butyl, n-Pentyl, n-Hexyl, Allyl, Propargyl, für Cyclohexyl, Cyclopentyl, Cyclohexylmethyl, Methylcyclohexyl, 4-Methylcyclohexylmethyl oder 3-Methylcyclohexylmethyl steht und

$R^2$ für Cyclohexyl, Phenyl oder einen der Reste

$-CH_2-R^3$,

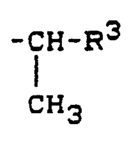 oder 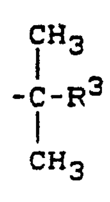

steht, wobei

$R^3$ jeweils für Wasserstoff, Methyl, Ethyl, n- oder i-Propyl, n-, i-, s- oder t-Butyl, Neopentyl, Cyclohexyl oder Phenyl steht.

**5.** Verfahren zur Herstellung von substituierten 1-Aminomethyl-3-aryl-4-cyano-pyrrolen der allgemeinen Formel (I),

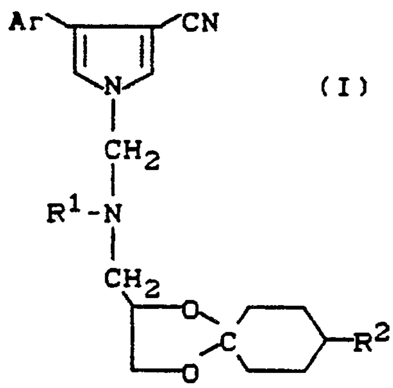

(I)

in welcher

Ar für gegebenenfalls substituiertes Phenyl steht,

$R^1$ für gegebenenfalls substituiertes Alkyl, für Alkenyl, Alkinyl oder Cycloalkyl oder für jeweils gegebenenfalls substituiertes Aralkyl oder Aryl steht und

$R^2$ für Alkyl oder für jeweils gegebenenfalls substituiertes Cycloalkylalkyl, Aralkyl, Cycloalkyl oder Aryl steht,

und deren Säureadditionssalze, Isomere und Isomerengemische, dadurch gekennzeichnet, daß man

(a) 3-Aryl-4-cyano-pyrrole der Formel (II),

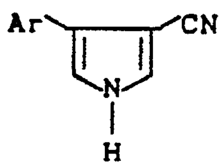

(II)

in welcher

Ar die oben angegebene Bedeutung hat,

mit Formaldehyd und Aminen der Formel (III),

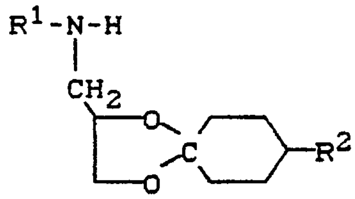

(III)

in welcher

$R^1$ und $R^2$ die oben angegebene Bedeutung haben,

gegebenenfalls in Gegenwart eines Verdünnungsmittels und gegebenenfalls in Gegenwart eines Reaktionshilfsmittels umsetzt, oder daß man

(b) 3-Aryl-4-cyano-pyrrole der Formel (IV),

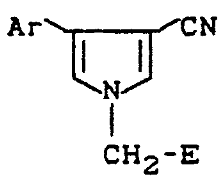

(IV)

in welcher

Ar die oben angegebene Bedeutung hat und

E für eine elektronenanziehende Abgangsgruppe steht,

mit Aminen der Formel (III),

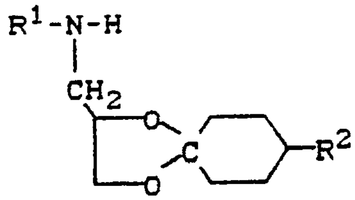

(III)

in welcher

R¹ und R² die oben angegebene Bedeutung haben,

gegebenenfalls in Gegenwart eines Verdünnungsmittels und gegebenenfalls in Gegenwart eines Säurebindemittels umsetzt und gegebenenfalls anschließend eine Säure addiert oder eine physikalische Trennmethode anschließt.

**6.** Schädlingsbekämpfungsmittel, gekennzeichnet, durch einen Gehalt an mindestens einem 1-Aminomethyl-3-aryl-4-cyano-pyrrol der Formel (I) nach den Ansprüchen 1 und 5.

**7.** Verwendung von 1-Aminomethyl-3-aryl-4-cyano-pyrrolen der Formel (I) nach den Ansprüchen 1 und 5 zur Bekämpfung von Schädlingen.

**8.** Vorfahren zur Bekämpfung von Schädlingen, dadurch gekennzeichnet, daß man 1-Aminomethyl-3-aryl-4-cyano-pyrrole der Formel (I) nach den Ansprüchen 1 und 5 auf Schädlinge und/oder ihren Lebensraum einwirken läßt.

**9.** Verfahren zur Herstellung von Schädlingsbekämpfungsmitteln, dadurch gekennzeichnet, daß man 1-Amino-methyl-3-aryl-4-cyano-pyrrole der Formel (I) nach den Ansprüchen 1 und 5 mit Streckmitteln und/oder oberflächenaktiven Mitteln vermischt.

**10.** Verwendung von 1-Aminomethyl-3-aryl-4-cyano-pyrrolen der Formel (I) gemäß Anspruch 7 im Pflanzenschutz und Materialschutz.

Europäisches Patentamt

# EUROPÄISCHER RECHERCHENBERICHT

Nummer der Anmeldung

EP 90123609.1

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl 5) |
|---|---|---|---|
| D,A | EP - A2 - 0 310 558 (CIBA-GEIGY) * Ansprüche 1,29-34 * -- | 1,6-9 | C 07 D 405/12<br>A 01 N 43/36 |
| A | EP - A1 - 0 327 977 (BAYER) * Ansprüche 1,8-11; Beispiele 1-4 * -- | 1,6-9 | |
| A | EP - A1 - 0 327 987 (BAYER) Ansprüche 1,8-11; Beispiele 1,2,3,7,9 * -- | 1,6-9 | |
| A | DE - A1 - 3 702 853 (BAYER) Ansprüche 1,6-9 -- | 1,6-9 | |
| A | DE - A1 - 3 702 852 (BAYER) Ansprüche 1,8-11; Beispiele 1,2,6,7 ---- | 1,6-9 | RECHERCHIERTE SACHGEBIETE (Int Cl 5)<br>C 07 D 405/00 |

Der vorliegende Recherchenbericht wurde für alle Patentansprüche erstellt.

| Recherchenort | Abschlußdatum der Recherche | Prüfer |
|---|---|---|
| WIEN | 01-02-1991 | HAMMER |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82